# EUROPEAN PATENT SPECIFICATION

(11) **EP 3 897 962 B1**
(45) Date of publication and mention of the grant of the patent: **12.11.2025**
(21) Application number: 19900943.2
(22) Date of filing: 23.12.2019
(51) Int. Cl.: B01J 19/08, B01J 35/00, B01J 27/00, B01J 19/12, B01J 21/06, B01J 23/02, B01J 23/10, B01J 37/34, C07C 29/159, C07C 51/00

(54) **METHODS AND PRODUCTS FOR CONVERTING CARBON DIOXIDE TO ONE OR MORE SMALL ORGANIC COMPOUNDS**
VERFAHREN UND PRODUKTE ZUR UMWANDLUNG VON KOHLENDIOXID IN EINE ODER MEHRERE KLEINE ORGANISCHE VERBINDUNGEN
PROCÉDÉS ET PRODUITS PERMETTANT DE CONVERTIR DU DIOXYDE DE CARBONE EN UN OU PLUSIEURS PETITS COMPOSÉS ORGANIQUES

(30) Priority: 21.12.2018 AU 2018904898
(43) Date of publication of application: 27.10.2021
(73) Proprietor: CarbonX Developments Pty Ltd, Adelaide SA 5000 (AU)
(72) Inventor: JONES, Bryn, Littlehampton, South Australia 5250 (AU); KELLY, Julian F., Robe, South Australia 5276 (AU)
(74) Representative: Müller, Christian Stefan Gerd
(86) International application number: PCT/AU2019/051431
(87) International publication number: WO 2020/124169

(56) References cited:
- WO-A1-2014/121121
- US-A- 5 022 970
- US-A1- 2011 011 728
- US-A1- 2015 375 192
- FLORIAN PONTZEN , WALDEMAR LIEBNER , VERONIKA GRONEMANN , MARTIN ROTHAEMEL ,BERND AHLERS: "C02-Based Methanol and DME - Efficient Technologies for Industrial Scale Production", CATALYSIS TODAY, vol. 171, no. 1, 2011, pages 242 - 250, XP055721694, ISSN: 0920-5861, DOI: 10.1016/j.cattod.2011.04.049

## Description

### FIELD

The present disclosure relates to methods, systems products for converting carbon dioxide to one or more small organic compounds.

### BACKGROUND

The production of carbon dioxide (CO₂) is widely considered to be a contributing factor to global warming. This has led to the recognition that it would be highly desirable to develop new technologies to prevent the accumulation of CO₂.

A variety of technologies have been developed to reduce net atmospheric emissions, including its removal through processes such as scrubbing or sequestration of the gas in highly porous materials. However, another means of reducing CO₂ is to convert the material into commercially useful compounds, for example, into high energy compounds such as methane which can themselves be used to generate power or into compounds that can be used in other commercial processes.

Processes for converting CO₂ into other commercially useful compounds are known, but these processes typically involve large amounts of input energy, rely on the use of other compounds, and/or are inefficient for large scale conversion of CO₂. For example, some photo catalytic conversion systems have been developed, but these remain inefficient.

Accordingly, it would be desirable to develop new processes for converting carbon dioxide into commercially useful compounds, particularly high energy compounds that may be used for generating power. WO 2014/121121 discloses a radiolytic electrochemical generator.

### SUMMARY

The present invention is defined by the claims. Any subject-matter falling outside the scope of the claims is provided for information only.

The present disclosure relates to methods and products for converting carbon dioxide to one or more small organic compounds.

In a first aspect, the present invention provides a method of converting CO₂ and/or a related form thereof to one or more small organic compounds, the method comprising exposing the CO₂ and/or the related form thereof to a beta particle activated high band-gap semiconductor and thereby converting the CO₂ and/or the related form thereof to the one or more small organic compounds, wherein the one or more small organic compounds comprises one or more of carbon monoxide, formaldehyde, methane, methanol, formic acid, ethanol, acetaldehyde, acetic acid, propanol, and isopropanol; and wherein the semiconductor has a conduction band edge energy of less than -0.15 volts, with respect to the standard hydrogen electrode.

Also disclosed is a method of producing one or more small organic compounds, the method comprising using a method as described herein to convert CO₂ and/or a related form thereof to the one or more small organic compounds.

In a second aspect, the present invention provides a system for converting CO₂ and/or a related form thereof to one or more small organic compounds using the method of the first aspect, the system comprising:
a source of CO₂ and/or a related form thereof;
a reaction container comprising a radioactive catalyst comprising a high band-gap semiconductor and a beta particle emitting radionuclide for exposure to the CO₂ and/or the related form thereof; and
means for extracting the one or more small organic compounds produced by exposure of the CO₂ and/or the related form thereof to the radioactive catalyst.

In a third aspect, the present invention provides a radiocatalytic material comprising a high band-gap semiconductor coupled with a beta particle emitting radionuclide, wherein the semiconductor has a conduction band edge energy of less than -0.15 volts, with respect to the standard hydrogen electrode.

Other embodiments are disclosed herein.

### BRIEF DESCRIPTION OF FIGURES

Certain embodiments are illustrated by the following figures. It is to be understood that the following description is for the purpose of describing particular embodiments only and is not intended to be limiting with respect to the description.
Figure 1 shows an experimental setup for a reaction in the presence of β-emitter and a high band-gap semiconductor.
Figure 2 shows an experimental setup for non-radioactive treatments.
Figure 3 shows PTFE vessel setup using an alternative ⁸⁹Sr methodology. PTFE lid has Swagelok fittings including pressure relief valve (right) and manual open/close valve (top). Gas manifold has pressure gauge (top), isolation valves and two way valve for introduction of CO₂ and vacuum.
Figure 4 shows the setup of active experiments during CO₂ loading using the alternative ⁸⁹Sr methodology.
Figure 5 shows the setup for gas sampling, showing two way inlet valve for CO₂ and vacuum (left) and Tedlar bag (for gas sampling) attached to manifold (right).

### DETAILED DESCRIPTION

The present disclosure relates, at least in part, to methods, systems and products for converting carbon dioxide to one or more small organic compounds.

The present disclosure is based, at least in part, on the recognition that a radiocatalysis system can be used to convert waste carbon dioxide to valuable organic compounds, such as methanol.

Without being bound by theory, it has been recognised that exposing certain high band-gap semiconductors to a flux of energetic beta particles (for example from beta particle emitting radionuclides) electronically excites the high band-gap semiconductor to an activated state capable of driving electron transfer to CO₂ molecules. Accordingly, it is possible to convert carbon dioxide at electronically excited semiconductor sites where the electronic excitation creates a high reduction potential.

Methods and products are described herein that have one or more combinations of advantages. For example, some of the advantages of some of the embodiments disclosed herein include one or more of the following: a new and/or improved method for converting CO₂ into commercially useful compounds; new and/or improved methods for converting CO₂ into chemical compounds that may be used to generate energy; new and/or improved methods for converting waste CO₂ into commercially useful organic compounds; the ability to utilise certain radioactive waste materials to convert CO₂ to small organic compounds; adding value to radioactive compounds formerly considered as waste products; converting a "green-house" compound into a source of commercially useful compounds; assisting with reducing release of CO₂ into the atmosphere; a method that can potentially contribute to reducing anthropogenic climate change; a method for utilising bicarbonate and/or carbonate feedstocks to produce new commercially useful compounds; obviating the use of emissions-intensive H₂ to produce certain organic compounds, thereby improving safety, providing economic benefits, and benefits to greenhouse credentials; to address one or more problems and/or to provide one or more advantages, or to provide a commercial alternative. Other advantages of certain embodiments of the present disclosure are also disclosed herein.

In one aspect, the present invention provides a method of converting CO₂ and/or a related form thereof to one or more small organic compounds to one or more small organic compounds, as defined in claim 1.

The term "CO₂" as used herein refers to carbon dioxide or one of its related forms, for example a form present in a solvated or solid state, such as HCO₃⁻, CO₃²⁻ or H₂CO₃, or a form of CO₂ complexed with another molecule, and includes within its scope radicals and radical ions of the aforementioned chemical entities, or complexes with other molecules.

In certain embodiments, the CO₂ is dissolved in an aqueous solution, CO₂ in a gaseous form, for example as a gas mixed with water vapour, the use of CO₂ dissolved in another solvent, or the use of liquid CO₂ itself.

In certain embodiments, the method is carried out in solution. In certain embodiments, the method is carried out in an aqueous solution or a substantially aqueous solution. In certain embodiments, the method is carried out in a non-aqueous solution. In certain embodiments, the method is carried out in a solvent or a mixed solvent, such as dioxane or dioxane and water. In certain embodiments, the method is carried out in a gaseous or vapour state. Methods and apparatus for conducting reactions in the aforementioned states are known in the art.

In certain embodiments, the method is carried out under conditions where the CO₂ is in the liquid state, alone or mixed with other substances. Methods and apparatus for conducting reactions in liquid CO₂ are known in the art.

In certain embodiments, the CO₂ comprises one or more of waste CO₂, atmospheric CO₂, liquid CO₂, sequestered CO₂, a source of CO₂ complexed with another agent, a bicarbonate, a carbonate, a carbonate ore, or a source of a related form of CO₂. Other sources of CO₂ are contemplated.

The term "small organic compound" as used herein refers to any compound having one or more carbon atoms and which are bonded to another carbon atom and/or to another element, such as hydrogen, oxygen or nitrogen. It will be appreciated that the term includes within its scope carbon monoxide (CO) which is sometimes not classified as an organic compound, and also includes within its scope ions, complexes, and radicals of carbon containing compounds.

The one or more small organic compounds comprise one of more of carbon monoxide (CO), methane, H₂CO (formaldehyde), CH₃OH (methanol), HCO₂H (formic acid or the anion thereof), CH₃CHO (acetaldehyde), CH₃CH₂OH (ethanol), CH₃CH₂COOH (acetic acid or the anion thereof), CH₃CH₂CH₂OH (propanol), or (CH₃)₂CHOH (isopropanol). Other types of small organic compounds are contemplated. Methods for identifying small organic compounds are known in the art.

**In** certain embodiments, the method further comprises purifying or extracting the one or more small organic compounds. Methods for purifying or extracting small organic compounds are known in the art, for example distillation and condensation, or differential adsorption.

A suitable semiconductor having a high band-gap and a conduction band edge energy sufficient to enable the reduction of CO₂ may be selected, wherein the semiconductor has a conduction band edge energy of less than -0.15 volts, with respect to the standard hydrogen electrode.

**In** certain embodiments, the high band-gap semiconductor has both the properties of a band-gap of at least 2.0 eV and a conduction band edge energy of less than -0.15 V (more negative than) relative to the standard hydrogen electrode. Methods are known in the art for determining the characteristics of semiconductors.

**In** certain embodiments, the high band-gap semiconductor has a band-gap of at least 2.6 eV. **In** certain embodiments, the high band-gap semiconductor has a band-gap of at least 3.1 eV. **In** certain embodiments, the high band-gap semiconductor has a band-gap of at least 3.2 eV. **In** certain embodiments, the high band-gap semiconductor has band gap of at least 3.4 eV. **In** certain embodiments, the high band-gap semiconductor has a band-gap in the range from 2.6 to 5.4 eV, 3.1 to 5.4 eV, 3.2 to 5.4 eV, or 3.4 to 5.4 eV.

**In** certain embodiments, the high band-gap semiconductor has a conduction band edge energy of less than (more negative than) -0.4 volts with respect to the standard hydrogen electrode. **In** certain embodiments, the high band-gap semiconductor has a conduction band edge energy of less than (more negative than) -0.8 volts with respect to the standard hydrogen electrode. In certain embodiments, the high band-gap semiconductor has a conduction band edge energy of less than (more negative than) -2.0 volts with respect to the standard hydrogen electrode.

Examples of high band-gap semiconductors include a titanate, zirconate, molybdate, vanadate, technetate, pertechnetate, tungstate, niobate, tantalate, doped tin oxides, doped zinc oxide, a hafnate, a germanium oxide, an oxide of manganese, cobalt and iron (eg a ferrate, a manganate, a cobaltate), a chromate, a simple oxide, a sulphide, a chalcogenide and a carbon allotrope. Other types of high-band semiconductors are contemplated. High band gap semiconductors are commercially available or may be produced by a method known in the art.

In certain embodiments, the high band-gap semiconductor comprises a titanate and/or a zirconate.

In certain embodiments, the high band-gap semiconductor comprises one or more of a strontium zirconate (SrZrO₃), a strontium titanate (SrTiO₃) and a titanium oxide.

In certain embodiments, the high band-gap semiconductor has one or more of the following preferred characteristics: a low electron-hole-pair recombination rate; a melting point of at least 250°C; resistance to oxidation; hardness; strength; resistance to impact fracture, erosion and/or abrasion. Methods for assessing the aforementioned characteristics are known in the art.

In certain embodiments, the high band-gap semiconductor comprises a characteristic of the ability to be fabricated into a free-flowing powder form that does not self-agglomerate.

In certain embodiments, the beta particle activated high band-gap semiconductor comprises beta particle activation via emission from a radionuclide. In this regard, it will be appreciated that the radionuclide may also be a radionuclide that decays to a beta-emitting radionuclide.

In certain embodiments, the radionuclide also emits gamma (γ) radiation and/or emits γ radiation from one of its decay products.

In certain embodiments, the radionuclide comprises one or more of ⁹⁰Sr, ⁹⁹Tc, ³H , ¹⁴C , ⁶³Ni, ¹³⁷Cs, ¹⁴⁷Pm , ¹⁵¹Sm, ^{121m}Sn, ¹⁵⁵Eu, ⁹³Zr , ²¹⁰Pb and ¹²⁶Sn. Sources of radionuclides are known in the art, such as being obtained commercially. Methods for producing radionuclides are also known in the art. Other beta particle emitting radionuclides are contemplated.

In certain embodiments, the radionuclide has one or more of the following preferred properties: (i) the radionuclide emits β-particles with energies in the range 1 - 100 kilo electron volts (keV); (ii) the radionuclide emits β-particles at a rate governed by a half-life in the range of 1-10 years, for example ~5 years (to minimise replacement periods); and (iii) the radionuclide is an isotope of an element with tractable chemical characteristics, such that the radionuclide can be readily loaded into the high band-gap semiconductor.

It will be appreciated that a single radioactive beta decay event can cause a cascade of secondary electrons extending tens of micrometers (µm) from the original decaying atom, and each of these is potentially capable of causing excited electronic states within the high band-gap semiconductor.

In certain embodiments, the radionuclide is a radionuclide that produces multiple β-particle emissions via its chain of decaying daughter radionuclides as it ultimately decays to a stable isotope/ nucleus, such as ⁹⁰Sr and ¹²⁶Sn.

In certain embodiments, activation of the high band-gap semiconductor comprises exposure to β-particles emitted from a radionuclide in contact with, located at a distance from, and/or coupled with the high band-gap semiconductor.

In certain embodiments the radionuclide is physically incorporated into the high band-gap semiconductor. In certain embodiments, the radionuclide is chemically incorporated into the high band-gap semiconductor.

A suitable amount of loading of the radionuclide into the high band-gap semiconductor may be selected. In certain embodiments, the high band-gap semiconductor is loaded with radionuclide in the range from 01.-100 GBq/mm³, 1.0-100 GBq/mm³, or 10-100 GBq/mm³. Other ranges are contemplated.

In certain embodiments, the radionuclide is proximal to the high band-gap semiconductor, physically admixed with the high band-gap semiconductor, chemically incorporated into the high band-gap semiconductor, present in a matrix of the high band-gap semiconductor, or located internally to the high band-gap semiconductor. Other arrangements are contemplated.

In certain embodiments, the high band-gap semiconductor and the radionuclide are coupled to form a radioactive catalyst. In this regard, the term "radioactive catalyst" as used herein may also be referred to as a "radiocatalytic material".

In certain embodiments, the radioactive catalyst is in macroscopic form, for example as granules, beads, a powder, or consolidated into a porous solid form such as a frit.

In certain embodiments, the radionuclide is distributed substantially homogeneously in the high band-gap semiconductor.

In certain embodiments, the radionuclide is distributed substantially heterogeneously in the high band-gap semiconductor. For example, there may be a radioactively doped zone at the centre of high band-gap semiconductor particles, with the outer rim having no radioisotope content.

In certain embodiments, the radioactive catalyst comprises the radionuclide coating all or part of the high band-gap semiconductor. In certain embodiments, the radioactive catalyst comprises the radionuclide encapsulated by the high band-gap semiconductor. In certain embodiments, the radioactive catalyst comprises the radionuclide physically admixed with the high-band gap semiconductor. In certain embodiments, the radioactive catalyst has a graded distribution of radionuclide within the high band gap semiconductor. In certain embodiments, the radioactive catalyst comprises the radionuclide loaded into the high band-gap semiconductor. In certain embodiments, the radioactive catalyst comprises the radionuclide chemically incorporated into the high band-gap semiconductor. In certain embodiments, the radioactive catalyst comprises a matrix comprising the radionuclide and the high band-gap semiconductor. Other arrangements are contemplated.

In certain embodiments, the radioactive catalyst comprises a radioactive content of 0.1 GBq/mm³ or greater. In certain embodiments, the radioactive catalyst comprises a radioactive content of 1 GBq/mm³ or greater. In certain embodiments, the radioactive catalyst comprises a radioactive content of 10 GBq/mm³ or greater. In certain embodiments, the radioactive catalyst comprises a radioactive content of 100 GBq/mm³ or greater.

In certain embodiments, the radioactive catalyst comprises a radioactivity content in the range from 0.1 - 100 GBq/mm³ range, 1.0 - 100 GBq/mm³, or 10 - 100 GBq/mm³. Other ranges are contemplated.

In certain embodiments, the radioactive catalyst has a high surface area. In certain embodiments, the radioactive catalyst has a surface area of 1 m²g⁻¹ or greater, 10 m²g⁻¹ or greater, or 100 m²g⁻¹ or greater. Methods for assessing surface area are known in the art.

In certain embodiments, the radioactive catalyst is in a macroscopic form. In certain embodiments, the radioactive catalyst is in a porous macroscopic form.

In certain embodiments, the radioactive catalyst is porous, having sufficient open porosity to permit a reactant fluid (liquid or gas) to enter pores and/or flow through the bulk catalyst without high applied pressure.

In certain embodiments, the radioactive catalyst is a composite material. For example, a dispersion of ¹⁴C particles (eg graphene, amorphous carbon, or diamond) in a semiconductor matrix.

In certain embodiments, the radioactive catalyst is a ceramic material. Methods for producing ceramics are known in the art.

In certain embodiments, the radioactive catalyst is a cermet material ("ceramic metal composite material"). Methods for producing such materials are known in the art.

In certain embodiments, the method of converting CO₂ and/or a related form thereof to one or more small organic compounds comprises exposing a high band-gap semiconductor undergoing electronic excitation by energetic beta-particles to the CO₂ or the related form thereof and thereby converting the CO₂ and/or the related form thereof to the one or more small organic compounds.

In certain embodiments, the method of converting CO₂ and/or a related form thereof to one or more small organic compounds comprises activating a high band-gap semiconductor by energetic β-particles emitted from a radionuclide and exposing the high band-gap semiconductor to the CO₂ and the related form thereof, and thereby converting the CO₂ and/or the related form thereof to the one or more small organic compounds.

In certain embodiments, the method of converting CO₂ and/or a related form thereof to one or more small organic compounds comprises exposing the CO₂ and/or the related form thereof to a beta particle emitting radionuclide coupled to a high band-gap semiconductor and thereby converting the CO₂ and/or the related form thereof to the one or more small organic compounds.

In certain embodiments, the method of converting CO₂ and/or a related form thereof to one or more small organic compounds comprises exposing the CO₂ and/or the related form thereof to a beta particle emitting radionuclide in the presence of a high band-gap semiconductor and thereby converting the CO₂ and/or the related form thereof to the one or more small organic compounds.

In certain embodiments, method of converting CO₂ and/or a related form thereof to one or more small organic compounds comprises exposing the CO₂ and/or the related form thereof to a high band-gap semiconductor activated by beta particles from a radionuclide and thereby converting the CO₂ and/or the related form thereof to the one or more small organic compounds.

Also disclosed is a method of producing one or more small organic compounds using a method as described herein to convert CO₂ and/or a related form thereof to the one or more small organic compounds.

Small organic compounds are as described herein.

In certain embodiments, the methods further comprise purifying or extracting the one or more small organic compounds. Methods for purifying or extracting small organic compounds are as described herein. Methods for determining the extent of purification/extraction are known in the art.

One or more small organic compounds can be produced by a method as described herein.

Examples of small organic compounds are as described herein. In certain embodiments, the small organic compound comprises one or more of carbon monoxide, formaldehyde, methane, methanol, formic acid, acetaldehyde, ethanol, acetic acid, propanol, and isopropanol.

Methods for purifying or extracting small organic compounds are known in the art. For example, the small organic compounds may be separated and purified using processes such as distillation or differential adsorption. Other methods are contemplated.

In a further aspect, the present invention provides a system for converting CO₂ and/or a related form thereof to one or more small organic compounds using the method of the first aspect, as defined in claim 10.

In certain embodiments, the reaction container comprises a high band-gap semiconductor coupled closely with a beta particle emitting radionuclide for exposure to the CO₂ and/or the related form thereof.

In certain embodiments, the reaction container comprises a high band-gap semiconductor coupled closely with a beta particle emitting radionuclide for exposure to the CO₂ and/or the related form thereof.

In certain embodiments, the source of CO₂ comprises one or more of waste CO₂, atmospheric CO₂, liquid CO₂, sequestered CO₂, CO₂ complexed with another agent, a bicarbonate, a carbonate, or a carbonate ore, or a chemical compound that provides CO₂. Other sources of CO₂ are contemplated.

In certain embodiments, the radioactive catalyst comprises a porous solid form through which reactant fluids can pass.

In certain embodiments, the reaction container comprises the radionuclide and the high band-gap semiconductor in a granular form in a fluidised bed in which the desired chemical reactions take place. In certain embodiments, the reaction container comprises the radioactive catalyst in a granular form in a fluidised bed in which the desired chemical reactions take place.

In certain embodiments, the means for extracting small organic molecules comprises a distillation means and/or a condensing means, or differential adsorption means. Other means for extracting small organic compounds are contemplated.

In certain embodiments, the system comprises a production plant for the production of one or more small organic compounds, for example methanol.

In certain embodiments, the systems and/or methods described herein are for producing one or more small organic compounds from CO₂ and/or a related form thereof.

Examples of small organic compounds are as described herein. In certain embodiments, the small organic compound comprises one or more of carbon monoxide, formaldehyde, methane, methanol, formic acid, acetaldehyde, ethanol, acetic acid, propanol, and isopropanol.

Also disclosed is a method of activating a high band-gap semiconductor.

In certain embodiments, the high-band gap semiconductors are suitable for conversion of CO₂ (and/or a related form thereof) to one or more small organic compounds. Other uses are contemplated.

A high band-gap semiconductor may be activated by a method as described herein.

**In** certain embodiments, the high band-gap semiconductor has both the properties of a band-gap of at least 2.0 eV and a conduction band edge energy of -0.15 V or less (more negative than) relative to the standard hydrogen electrode.

**In** certain embodiments, the high band-gap semiconductor has a band-gap of at least 2.6 eV. **In** certain embodiments, the high band-gap semiconductor has a band-gap of at least 3.1 eV. **In** certain embodiments, the high band-gap semiconductor has a band-gap of at least 3.2 eV. **In** certain embodiments, the high band-gap semiconductor has band gap of at least 3.4 eV. **In** certain embodiments, the high band-gap semiconductor has a band-gap in the range from 2.6 to 5.4 eV, 3.1 to 5.4 eV, 3.2 to 5.4 eV, or 3.4 to 5.4 eV.

In certain embodiments, the high band-gap semiconductor has a conduction band edge energy of less than (more negative than) -0.15 volts, with respect to the standard hydrogen electrode.

In certain embodiments, the high band-gap semiconductor has a conduction band edge energy of less than (more negative than) -0.4 volts with respect to the standard hydrogen electrode. In certain embodiments, the high band-gap semiconductor having a conduction band edge energy of less than (more negative than) -0.8 volts with respect to the standard hydrogen electrode. In certain embodiments, the high band-gap semiconductor has a conduction band edge energy of less than (more negative than) -2.0 volts with respect to the standard hydrogen electrode.

Also disclosed is a method of producing an activated high band-gap semiconductor.

In certain embodiments, the activated high band-gap semiconductor is suitable for the conversion of CO₂ and/or a related form thereof to one or more small organic compounds. Other uses are contemplated.

Also disclosed is a method of producing an activated high band-gap semiconductor, the semiconductor having a conduction band edge energy sufficient to enable the reduction of CO₂, the method comprising exposing the semiconductor to a beta particle emitting radionuclide and thereby producing the activated high band-gap semiconductor.

An activated high band-gap semiconductor may be produced by a method as described herein.

In a further aspect, the present invention provides a radiocatalytic material comprising a high band-gap semiconductor coupled with a beta particle emitting radionuclide, as defined in claim 12.

In certain embodiments, the radiocatalytic material comprises a high band-gap semiconductor loaded with a beta particle emitting radionuclide, wherein the semiconductor is as defined in claim 12.

Radiocatalytic materials comprising a high band-gap semiconductor coupled to a beta particle emitting radionuclide are as described herein.

In certain embodiments, the radioactive catalyst material comprises a radioactive content of 0.1 GBq/mm³ or greater. In certain embodiments, the radioactive catalyst material comprises a radioactive content of 1.0 GBq/mm³ or greater. In certain embodiments, the radioactive catalyst material comprises a radioactive content of 10 GBq/mm³ or greater. In certain embodiments, the radioactive catalyst comprises a radioactive content of 100 GBq/mm³ or greater. In certain embodiments, the radioactive catalyst material comprises a radioactivity content in the range from 0.1-100 GBq/mm³1.0-100 GBq/mm³, or 10-100 GBq/mm³. Other ranges are contemplated.

In certain embodiments, the radiocatalytic material comprises a beta particle emitting radionuclide encapsulated by a high band-gap semiconductor, wherein the semiconductor is as defined in claim 12.

Radiocatalytic materials comprising a beta particle emitting radionuclide encapsulated by a high band-gap semiconductor are as described herein.

High band-gap semiconductors and beta particle emitting radionuclides are as described herein.

In certain embodiments, the high band-gap semiconductor has both the properties of a band-gap of at least 2.0 eV and a conduction band edge energy of -0.15 V or less (more negative than) relative to the standard hydrogen electrode.

In certain embodiments, the high band-gap semiconductor has a band-gap of at least 2.6 eV. In certain embodiments, the high band-gap semiconductor has a band-gap of at least 3.1 eV. In certain embodiments, the high band-gap semiconductor has a band-gap of at least 3.2 eV. In certain embodiments, the high band-gap semiconductor has band gap of at least 3.4 eV. In certain embodiments, the high band-gap semiconductor has a band-gap in the range from 2.6 to 5.4 eV, 3.1 to 5.4 eV, 3.2 to 5.4 eV, or 3.4 to 5.4 eV.

The high band-gap semiconductor comprised in the radiocatalytic materials has a conduction band edge energy of less than (more negative than) -0.15 volts, with respect to the standard hydrogen electrode.

In certain embodiments, the high band-gap semiconductor has a conduction band edge energy of less than (more negative than) -0.4 volts with respect to the standard hydrogen electrode. In certain embodiments, the high band-gap semiconductor having a conduction band edge energy of less than (more negative than) -0.8 volts with respect to the standard hydrogen electrode. In certain embodiments, the high band-gap semiconductor has a conduction band edge energy of less than (more negative than) -2.0 volts with respect to the standard hydrogen electrode.

Also disclosed is the use of a radiocatalytic material as described herein for producing one or more small organic compounds from CO₂ and/or a related form thereof.

In certain embodiments, the radiocatalytic material is porous in form. In certain embodiments, the radiocatalytic material is in a form comprising a particle, a granule, a bead, a powder, or a pellet.

In certain embodiments, the radionuclide is distributed substantially homogeneously within the high band-gap semiconductor.

In certain embodiments, the radionuclide is distributed substantially heterogeneously within the high band-gap semiconductor. For example, a radioactively doped zone at the centre of a radiocatalyst particle and the outer rim has no radioisotope loading.

Also disclosed is the use of a radiocatalytic material for stimulating the production of one or more small organic compounds from CO₂ and/or a related form thereof.

Also disclosed is a composition comprising a solution of dissolved CO₂ and/or a related form thereof, a beta particle emitting radionuclide and a high band-gap semiconductor.

In certain embodiments, the composition is an aqueous composition, a gaseous composition or a liquid composition, including a liquid solvent or liquid gas.

Also disclosed is a composition comprising a solution of dissolved CO₂ and/or a related form thereof and a radiocatalytic material comprising a beta particle emitting radionuclide and a high band-gap semiconductor.

Also disclosed is a method of identifying a high band-gap semiconductor for converting CO₂ and/or a related form thereof to one or more small organic compounds by beta particle activation of the semiconductor.

Also disclosed is a method of identifying a high band-gap semiconductor for converting CO₂ and/or a related form thereof to one or more small organic compounds by beta particle activation of the semiconductor, the method comprising:
exposing CO₂ and/or a related form thereof to a beta particle emitting radionuclide coupled closely with a candidate high band-gap semiconductor; and
determining the ability of the candidate high band-gap semiconductor to convert the CO₂ and/or the related form thereof to one or more small organic compounds, thereby identifying the candidate high band-gap semiconductor as a high band-gap semiconductor for converting CO₂ and/or a related form thereof to one or more small organic compound by beta particle activation of the semiconductor.

**In** certain embodiments, the high band-gap semiconductor has both the properties of a band-gap of at least 2.0 eV and a conduction band edge energy of -0.15 V or less (more negative than) relative to the standard hydrogen electrode.

**In** certain embodiments, the high band-gap semiconductor has a band-gap of at least 2.6 eV. **In** certain embodiments, the high band-gap semiconductor has a band-gap of at least 3.1 eV. **In** certain embodiments, the high band-gap semiconductor has a band-gap of at least 3.2 eV. **In** certain embodiments, the high band-gap semiconductor has band gap of at least 3.4 eV. **In** certain embodiments, the high band-gap semiconductor has a band-gap in the range from 2.6 to 5.4 eV, 3.1 to 5.4 eV, 3.2 to 5.4 eV, or 3.4 to 5.4 eV.

In certain embodiments, the high band-gap semiconductor has a conduction band edge energy of less than (more negative than) -0.15 volts, with respect to the standard hydrogen electrode.

In certain embodiments, the high band-gap semiconductor has a conduction band edge energy of less than (more negative than) -0.4 volts with respect to the standard hydrogen electrode. In certain embodiments, the high band-gap semiconductor has a conduction band edge energy of less (more negative than) than -0.8 volts with respect to the standard hydrogen electrode. In certain embodiments, the high band-gap semiconductor has a conduction band edge energy of less than (more negative than) -2.0 volts with respect to the standard hydrogen electrode.

Examples of candidate semiconductors include a titanate, zirconate, molybdate, vanadate, technetate, pertechnetate, tungstate, niobate, tantalate, doped tin oxides, doped zinc oxide, a hafnate, an oxide of manganese, cobalt and iron (eg a ferrate, a manganate, a cobaltate), a chromate, a germanium oxide, a simple oxide, a sulphide, a chalcogenide and a carbon allotrope. In certain embodiments, the high band-gap semiconductor comprises a titanate and/or a zirconate.

Also disclosed is a high band-gap semiconductor for converting CO₂ and/or a related form thereof to a small organic compound by beta particle activation identified by a method as described herein.

The present disclosure is further described by the following examples. It is to be understood that the following description is for the purpose of describing particular embodiments only and is not intended to be limiting with respect to the above description.

### EXAMPLE 1 - Transformation of carbon dioxide to carbon products using radioactive Yttrium-90 (a β-Particle Emitter)

### 1. Introduction

A proof-of-concept study was undertaken to demonstrate the conversion of carbon dioxide (CO₂) to one or more small organic compounds, such as methanol, ethanol, propanol or formic acid.

Photocatalysis may be used to convert CO₂ to other compounds. These type of techniques rely on light energy sources to reduce CO₂ to other compounds by pairing the light energy (usually UV light) with a catalytic material to break the C=O bond in CO₂ in a H₂O environment. There are a number of reaction conditions which may influence the products formed including, catalyst type, light source and pH. However, one of the key limitations of the process is the need to use a light source providing a reasonable flux of photons having an energy greater than the photocatalytic semiconductor band-gap.

It was recognised that an alternative to a light source would be to utilise the energy of particles released during radioactive decay to energise the catalyst. In a proof-of-concept study, yttrium-90 (⁹⁰Y) was selected as a source of beta particles, in conjunction with a strontium titanate (SrTiO₃) catalyst, to investigate the transformation of CO₂ to organic compounds over a 6 week period. At the end of the study, the liquid samples were analysed for the presence of various small organic compounds.

### 2. Materials and Methods

### (i) Reaction in Presence of β-Emitter

The proof-of-concept studies were designed around the availability and form of a special beta radiation source - a multitude of functionalised polystyrene resin microbeads (18 - 30 µm diameter) loaded with the yttrium-90 (⁹⁰Y) isotope, referred to as "⁹⁰Y-loaded polymer microspheres". Such beads are available and used as a radio-embolization therapy for controlling metastatic liver tumours.

The experimental arrangement involved agitating the beta-emitting resin beads together with SrTiO₃ semiconductor particles in a related HCO₃⁻ (bicarbonate) solution as a source of soluble carbon dioxide. Such solutions 'self buffer' to a pH of around 8.15.

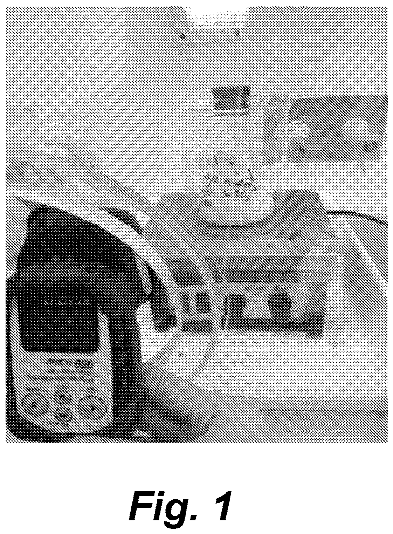

The activity of the ⁹⁰Y used was between 1.1 and 2.2 GBq. Upon receipt of the material, the ⁹⁰Y-loaded polymer microspheres were removed from the container and added to an Erlenmeyer flask containing a 100 mL solution of NaHCO₃ (40 g L⁻¹) and suspended SrTiO₃ powder (5 g L⁻¹). The solution was stirred at a constant speed to maintain a small vortex in the solution surface. The experimental setup is shown in Figure 1. The experiment was conducted behind a 10-mm perspex shield and the exposure dose monitored and recorded throughout the experimental period. The experiment was conducted in a radiation approved facility over a 6 week period and at the end of this period, samples were collected and filtered through 0.22 µm cellulose filters to remove the suspended materials. The gamma dose rate was monitored and recorded periodically throughout the experiment. Aqueous samples were analysed by an accredited external laboratory.

### (ii) Reaction in Absence of β-Emitter

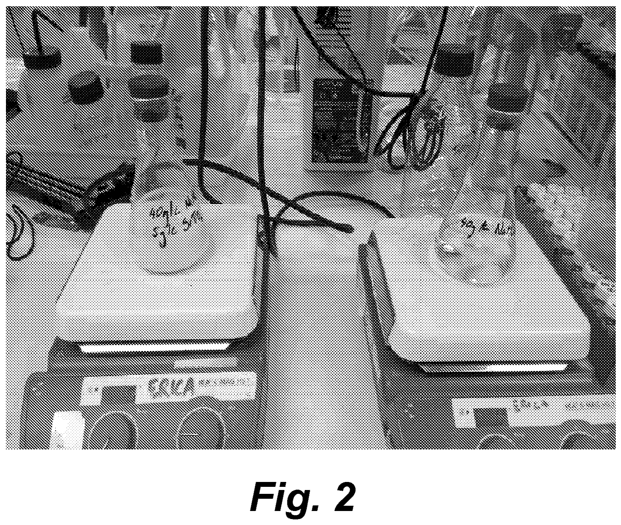

Non-radioactive "blank" reaction vessels (NaHCO₃ only and NaHCO₃ plus SrTiO₃) were set up as controls and used identical conditions as outlined for the ⁹⁰Y study (Figure 2). The experiment was run in parallel with the ⁹⁰Y experiment and samples were collected at the same time and filtered through 0.22 µm cellulose filters to remove the suspended materials. The samples were analysed by the accredited external laboratory.

### 3. Results

Analyses were made for the presence of methanol (CH₃OH), ethanol (CH₃CH₂OH), propanol, (CH₃ CH₂CH₂OH) and formic acid (HCOOH) in each of the three reaction environments, *ie,* the vessels with the beta-emitter present, and the blanks. The levels of other organic compounds - notably; methane (CH₄), carbon monoxide (CO) and formaldehyde (HCHO) were not measured in this experiment, as they would have been difficult to capture in the experimental arrangement.

**Table 1. Organic products identified**

| **Treatments** | **Products Identified** | | | |
|---|---|---|---|---|
| | Methanol (mg L⁻¹) | Ethanol (µg L⁻¹) | Propanol (µg L⁻¹) | Formic Acid (µg L⁻¹) |
| 0.5 M NaHCO₃ | <LOR | <LOR | <LOR | 487 |
| 0.5 M NaHCO₃ + SrTiO₃ | <LOR | <LOR | <LOR | 418 |
| 0.5 M NaHCO₃ + SrTiO₃ + ⁹⁰Y | 2.4 | <LOR | <LOR | 785 |

| | | | | |
|---|---|---|---|---|
| LOR for methanol is 1 mg 1⁻¹, and 50 µg L⁻¹ for ethanol, propanol and formic acid | | | | |

The assays showed that formic acid was present in all samples irrespective of the treatment (Table 1). However, treatments that did not include ⁹⁰Y contained approximately half the formic acid concentration compared to the treatment where ⁹⁰Y was added (Table 1). The formic acid concentration in the two treatments that did not include ⁹⁰Y were 418 and 487 mg L⁻¹, compared to a formic acid concentration of 785 mg L⁻¹ in the ⁹⁰Y treatment.

The presence of ethanol and propanol was not identified in any of the treatments (Table 1), but methanol was identified in the treatment which included ⁹⁰Y. Methanol was not identified in the other two non-radioactive beta radiation driven treatments (Table 1).

These studies demonstrate the conversion of CO₂ to small organic compounds such as methanol and formate by exposing a related form of CO₂ to a high band-gap semiconductor activated by a beta particle emitting radionuclide, such as ¹⁴C, ⁹⁰Sr, ⁹⁹Tc, ³H, ⁶³Ni, ¹³⁷Cs, ¹⁴⁷Pm , ¹⁵¹Sm, ^{121m}Sn, ¹⁵⁵Eu, ⁹³Zr , ²¹⁰Pb and ¹²⁶Sn.

### EXAMPLE 2 - Compositions for catalytic conversion of CO₂ to small organic compounds

A beta particle emitting radionuclide may be obtained from sources such as those where the radionuclide is regarded as waste or a liability, an example being nuclear industry processing facilities, where notable radionuclides are ¹⁴C, ⁹⁰Sr, ⁹⁹Tc, ³H, and ¹³⁷ Cs. These nuclides are typically isolated from aqueous process streams such as adsorbed salt species.

In one embodiment, the beta-emitting radionuclide may be exchanged for a similar cation in a high band-gap semiconductor such as strontium titanate (commercially available from chemical suppliers), using for example, an established hydrothermal process in a suitable autoclave which are known in the art. The resulting radioactive solid will be processed into a high surface area form (eg, a powder, granules, frit) such that it may be easily contacted with a solution containing dissolved CO₂ to produce small organic compounds such as carbon monoxide, methane or methanol.

In another embodiment, solid titanate/zirconate semiconductors may be loaded with a beta-emitting isotope using methods such as (i) 'solvothermal' approaches in which precursor oxides are reacted together in a high temperature aqueous fluid (up to ~240°C), typically with high pH, for example using a modification of the method as described in Modeshia and Walton (2010) Chemical Society Reviews 39:4303-4325; (ii) solid state reaction approaches in which powders of precursor phases are blended together and raised to a high temperature at which desired structural transformations and consolidation take place, for example using a modification of the method as described in Fu et al. (2010) Physica Scripta T139:1-4.

In a further embodiment, solid titanate/zirconate semiconductors may be loaded with a beta-emitting isotope using high energy physical mixing approaches to transform constituent oxides into desired titanates or zirconates, which are known in the art.

In another embodiment, hollow porous strontium titanate particles containing the beta particle emitting radionuclide may be produced by adapting the method as described in Tzeng and Shih (2015) Journal of the American Ceramic Society 98(2): 386-391. This method permits the production of porous powders with a high surface area, which may be contacted with a solution such as one containing dissolved CO₂, serving as a parent fluid for the radiocatalytic production of small organic compounds such as methanol.

### EXAMPLE 3- Production of small organic compounds such as methanol

The present disclosure relates to technology in which the energy of certain radioactive particles is harnessed to achieve a number of useful industrial endpoints. The study in Example 1 has demonstrated the feasibility of a radiocatalysis system for converting waste carbon dioxide to valuable organic compounds. The carbon dioxide may be, for example, waste carbon dioxide and/or a bicarbonate feedstock.

In some embodiments, the present disclosure utilises a high band-gap semiconductor into which a suitable radioactive isotope with suitable particle-emitting characteristics can be intrinsically incorporated (eg doped), such that the radioactivity content of the semiconductor is in a range, for example, in the range from 1.0 - 100 GBq /mm³ range.

In some embodiments, the radioisotope may be distributed heterogeneously within the solid semiconductor particle, for example where the radioactively doped zone is at the centre of a particle and the outer rim has no radioisotope loading.

It is envisaged that the material may be used in various physical forms, including powder, granules, or as a porous 'frit'.

The system may use a chemical reaction vessel which (i) contains physical radioactive catalyst arranged in a manner that leads to a high degree of contact between the catalyst surface and a related solution containing dissolved carbon dioxide, (ii) possesses radiation shielding measures to prevent occupational radiation doses from the static physical catalyst residing within the vessel; and (iii) delivers its outflow fluids to a supplementary organic compound separation system.

It is envisaged that the physical catalyst may be in a porous solid form through which reactant fluids can pass, or a granular form and which can be used to create a fluidised bed in which the desired chemical reactions may take place.

In some embodiments, the catalyst is selected from either or both strontium zirconate (SrZrO₃) and strontium titanate (SrTiO₃). These compounds are significant because they have a high band-gap (>2.0 eV), a conduction band edge energy that leads to a strong electrochemical reduction potential for excited electron hole pairs, and there is a strontium isotope (⁹⁰Sr) which is has excellent beta-particle emitting properties in terms of its half-life and energy.

A proposed system for the large scale conversion of CO₂ using the catalytic activity of a beta particle activated semiconductor is described below.

A solution of concentrated CO₂, CO₃²⁻ or HCO₃⁻ may be fed continuously into a reaction chamber comprising a slurry with a composition as described in Example 2, and which is mixed by constant agitation with a residence time in the order of hours, which catalytically converts the CO₂ into a number of small organic compounds, including methanol.

A portion of the aqueous solution is drawn off periodically and subject to mild centrifugation and/or filtration to separate suspended solids from the solution, and the remaining aqueous solution fed into a series of distillation chambers and condensing chambers, which allows production of high grade methanol. Methods for producing methanol by distillation are known in the art, for example as described in WO 2013/110368.

### EXAMPLE 4 - Use of ⁸⁹Sr in conjunction with strontium titanate

### 1. Methodology

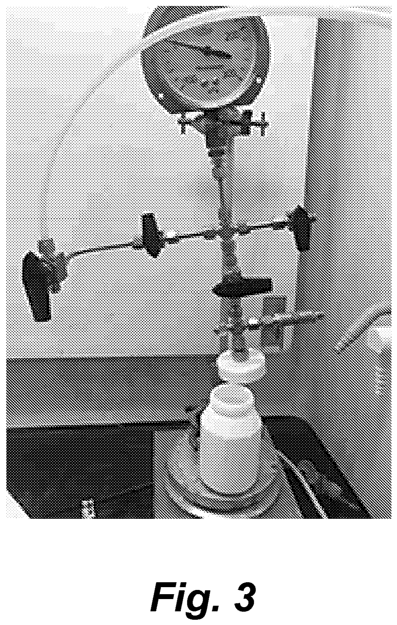

For the non-radioactive "blank" reaction, strontium titanate (SrTiO₃, 1.99 g) was added to a 250 mL PTFE vessel followed by addition of 1,4-dioxane (97 mL), MilliQ water (3 mL) and a stirrer bar. The PTFE vessel lid, modified with Swagelok fittings including a pressure relief valve and manual open/close valve, was attached to a gas manifold with a pressure gauge and connected to a CO₂ gas bottle via a two way valve, as shown in Figure 3. A flow of CO₂ gas was passed through the manifold and through the PTFE lid to purge the air from the PTFE vessel as it was screwed onto the lid.

Once attached to the gas manifold, the PTFE vessel was loaded with CO₂ via the following method:
- CO₂ added to the reaction vessel until the pressure gauge indicated 3 atm.
- CO₂ inlet valve then closed.
- With stirring, CO₂ dissolved in the solvent and pressure decreased, as monitored by the pressure gauge.
- When the rate of pressure decrease slowed, pressure value was recorded and CO₂ valve opened to repeat the process.

In this way approximately 11 atm CO₂ was added to the reaction vessel over 10 minutes, at which point the rate of pressure decrease was very slow and the system was assumed to be close to equilibrium. The valve to the vessel was then closed, CO₂ in the manifold vented and the vessel disconnected from the manifold.

Once loaded with CO₂, the reaction vessel was placed on a stirrer plate and continued stirring (at approximately 600 rpm) for 17 days.

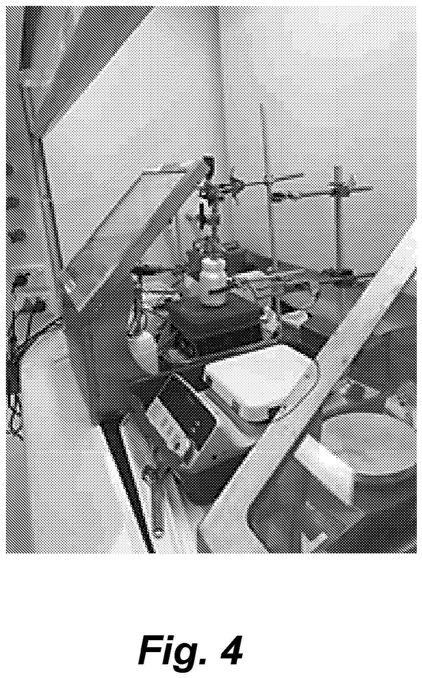

For the active reaction, identical reaction conditions were used, but the SrTiO₃ (2.1 g) was irradiated in the OPAL reactor for 9 days followed by 16 days decay. Gamma spectroscopy of the irradiated SrTiO₃ indicated a Sr-85 activity of 48.8 ± 4.4 MBq and hence a Sr-89 activity of 88 ± 21 MBq. The amount of CO₂ gas loaded into this reaction vessel was not explicitly measured but assumed to be similar to the inactive experiment (11 atm), as the reaction vessel was equilibrated via stirring under CO₂ at 3 atm for 15 min. CO₂ loading was performed behind lead shielding as shown in Figure 4, and the exposure dose was monitored and recorded throughout the experimental period.

Every 3-4 days the reaction vessels were re-attached to the gas manifold and reloaded with CO₂, as the pressure was observed to be dropping over time. The manifold was purged with CO₂ before being opened to the reaction vessels, to prevent contamination of the headspace gas with air. The measured pressures and volumes of CO₂ added at each time interval are given in Table 2.

**Table 2: Measured pressure (P, atm) and amount of CO₂ added (atm) over time for active and inactive experiments.**

| | **Day 4** | | **Day 7** | | **Day 8** | | **Day 11** | | **Day 14** | | **Day 17** | |
|---|---|---|---|---|---|---|---|---|---|---|---|---|
| | P | CO₂ | P | CO₂ | P | CO₂ | P | CO₂ | P | CO₂ | P | CO₂ |
| **active** | 1.0 | 1.5 | | | 0.6 | 4.6 | 0.1 | 5.4 | 0.1 | 5.2 | 0.4 | |
| **inactive** | 0 | 7.2 | 2.0 | 2.6 | | | 1.8 | 3.2 | 1.2 | 5.0 | 1.5 | |

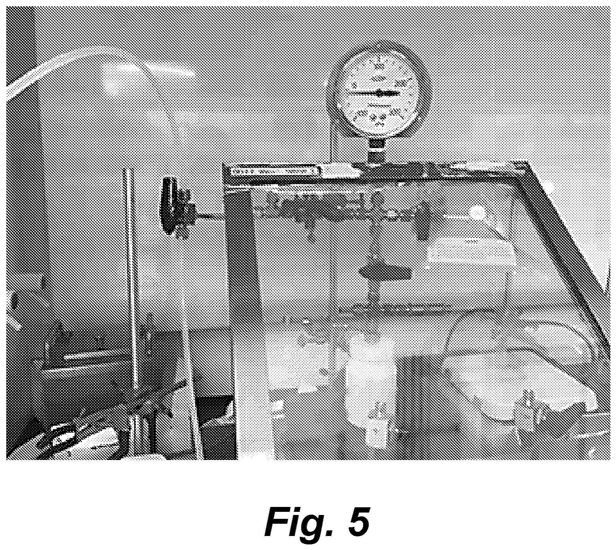

At the end of the 17 day experimental period, the headspace gas of each experiment (active and inactive) was sampled via the following method:
- Reaction vessel attached to the gas manifold.
- Tedlar bag (for gas sampling) attached to right hand side of manifold (see Figure 3).
- Manifold evacuated via vacuum pump attached to two way valve (see Figure 5).
- Reaction vessel opened to evacuated manifold, to record pressure.
- Valve to Tedlar bag opened until bag full.
- Sealed Tedlar bag removed from manifold and remaining CO₂ overpressure vented into fume hood.

The reaction vessel lid was then opened and the slurry within allowed to settle for 1.5 h. The supernatant liquid was sampled via pipette (2 mL) and centrifuged (5000 rpm, 5 min) to remove fines.

### 2. Results

Gas samples (active and inactive) were analysed for CO and CH₄ by an accredited external laboratory. Liquid samples (active and inactive) were analysed for methanol, ethanol, formaldehyde and formic acid via GC-MS. GC-MS was undertaken using an Agilent GC3800 with split injection mode (10:1), AT-WAX column with length 30 m, 0.32 mm I.D., df=0.5 µm, injection volume of 1 µL and MS1200 with electron ionisation, ionisation voltage 70 V and emission current 150 µA.

Levels of the following molecules will be determined: carbon monoxide, methane, methanol, ethanol, formaldehyde, and formic acid.

It is anticipated that the above reaction methodology will result in the production of a variety of small organic molecules.

Although the present disclosure has been described with reference to particular examples, it will be appreciated by those skilled in the art that the disclosure may be embodied in many other forms.

It is to be understood that various alterations, additions and/or modifications may be made to the parts previously described without departing from the ambit of the present disclosure, and that, in the light of the above teachings, the present disclosure may be implemented in software, firmware and/or hardware in a variety of manners as would be understood by the skilled person.

As used herein, the singular forms "a," "an," and "the" may refer to plural articles unless specifically stated otherwise.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

All methods described herein can be performed in any suitable order unless indicated otherwise herein or clearly contradicted by context. The use of any and all examples, or exemplary language (e.g., "such as") provided herein, is intended merely to better illuminate the example embodiments and does not pose a limitation on the scope of the claimed invention unless otherwise claimed. No language in the specification should be construed as indicating any non-claimed element as essential.

The description provided herein is in relation to several embodiments which may share common characteristics and features. It is to be understood that one or more features of one embodiment may be combinable with one or more features of the other embodiments. In addition, a single feature or combination of features of the embodiments may constitute additional embodiments.

The subject headings used herein are included only for the ease of reference of the reader and should not be used to limit the subject matter found throughout the disclosure or the claims. The subject headings should not be used in construing the scope of the claims or the claim limitations.

## Claims

1. A method of converting CO₂ and/or a related form thereof to one or more small organic compounds, the method comprising exposing the CO₂ and/or the related form thereof to a beta particle activated high band-gap semiconductor and thereby converting the CO₂ and/or the related form thereof to the one or more small organic compounds;
wherein the one or more small organic compounds comprises one or more of formaldehyde, methane, methanol, formic acid, ethanol, acetaldehyde acetic acid, propanol, and isopropanol; and
wherein the semiconductor has a conduction band edge energy of less than -0.15 volts, with respect to the standard hydrogen electrode.

2. The method according to claim 1, wherein the semiconductor has a band-gap of at least 2.6 eV.

3. The method according to claim 1 or claim 2, wherein the semiconductor comprises one or more of a titanate, zirconate, molybdate, vanadate, technetate, pertechnetate, tungstate, niobate, tantalate, chromate, doped tin oxides, doped zinc oxide, a hafnate, a germanium oxide, a simple oxide, an oxide of manganese, cobalt and iron, a sulphide, a chalcogenide and a carbon allotrope.

4. The method according to any one of claims 1 to 3, wherein the beta particle activated high band-gap semiconductor comprises beta particle activation via continuous excitation by beta particles.

5. The method according to any one of claims 1 to 4, wherein the beta particle activated semiconductor comprises beta particle activation via emission from a radionuclide.

6. The method according to claim 5, wherein the radionuclide comprises one or more of ¹⁴C, ⁹⁰Sr, ⁹⁹Tc, ³H, ⁶³Ni, ¹³⁷Cs, ¹⁴⁷Pm , ¹⁵¹Sm, ^{121m}Sn, ¹⁵⁵Eu, ⁹³Zr , ²¹⁰Pb and ¹²⁶Sn.

7. The method according to claims 5 or 6, wherein the beta particle activated semiconductor and the radionuclide are coupled to form a radioactive catalyst.

8. The method according to claim 7, wherein the radioactive catalyst comprises the radionuclide in contact with the high band-gap semiconductor, the radionuclide proximal to the high band-gap semiconductor, the radionuclide physically admixed with the high band-gap semiconductor, the radionuclide chemically incorporated into the high band-gap semiconductor, the radionuclide loaded into the high band-gap semiconductor, and/or the high band-gap semiconductor is located externally to the radionuclide.

9. The method according to claim 7 or 8, wherein the radioactive catalyst is in the form of a particle, a granule, a bead, a powder, a pellet or a frit.

10. A system for converting CO₂ and/or a related form thereof to one or more small organic compounds using the method according to any one of claims 1 to 9, the system comprising:
a source of CO₂ and/or a related form thereof;
a reaction container comprising a radioactive catalyst comprising a high band-gap semiconductor and a beta particle emitting radionuclide for exposure to the CO₂ and/or the related form thereof; and
means for extracting one or more small organic compounds produced by exposure of the CO₂ and/or the related form thereof to the radioactive catalyst.

11. The system according to claim 10, wherein the source of CO₂ and/or related form thereof comprises one or more of waste CO₂, atmospheric CO₂, liquid CO₂, sequestered CO₂, CO₂ complexed with another agent, a bicarbonate, a carbonate, a carbonate ore, or a chemical compound that provides CO₂.

12. A radiocatalytic material comprising a high band-gap semiconductor coupled with a beta particle emitting radionuclide, wherein the semiconductor has a conduction band edge energy of less than -0.15 volts, with respect to the standard hydrogen electrode.

## Patentansprüche

1. Verfahren zum Umwandeln von CO₂ und/oder einer verwandten Form davon in eine oder mehrere kleine organische Verbindungen, wobei das Verfahren ein Aussetzen des CO₂ und/oder der verwandten Form davon gegenüber einem durch beta-Partikel aktivierten Halbleiter mit hoher Bandlücke und dadurch Umwandeln des CO₂ und/oder der verwandten Form davon in die eine oder mehreren kleinen organischen Verbindungen umfasst;
wobei die eine oder mehreren kleinen organischen Verbindungen eines oder mehrere aus Formaldehyd, Methan, Methanol, Ameisensäure, Ethanol, Acetaldehyd, Essigsäure, Propanol und Isopropanol umfassen; und
wobei der Halbleiter eine Leitungsbandkantenenergie von weniger als -0,15 Volt, bezogen auf die Standard-Wasserstoffelektrode, aufweist.

2. Verfahren gemäß Anspruch 1, wobei der Halbleiter eine Bandlücke von mindestens 2,6 eV aufweist.

3. Verfahren gemäß Anspruch 1 oder Anspruch 2, wobei der Halbleiter eines oder mehrere aus einem Titanat, Zirkonat, Molybdat, Vanadat, Technetat, Pertechnetat, Wolframat, Niobat, Tantalat, Chromat, dotierten Zinnoxiden, einem dotierten Zinkoxid, einem Hafnat, einem Germaniumoxid, einem einfachen Oxid, einem Oxid von Mangan, Kobalt und Eisen, einem Sulfid, einem Chalkogenid und einem Kohlenstoffallotrop umfasst.

4. Verfahren gemäß einem jeglichen der Ansprüche 1 bis 3, wobei der durch beta-Partikel aktivierte Halbleiter mit hoher Bandlücke eine beta-Partikel-Aktivierung durch kontinuierliche Anregung durch beta-Partikel umfasst.

5. Verfahren gemäß einem jeglichen der Ansprüche 1 bis 4, wobei der durch beta-Partikel aktivierte Halbleiter eine beta-Partikel-Aktivierung durch Emission von einem Radionuklid umfasst.

6. Verfahren gemäß Anspruch 5, wobei das Radionuklid eines oder mehrere aus ¹⁴C, ⁹⁰Sr, ⁹⁹Tc, ³H, ⁶³Ni, ¹³⁷Cs, ¹⁴⁷Pm, ¹⁵¹Sm, ¹²¹Sn, ¹⁵⁵Eu, ⁹³Zr, ²¹⁰Pb und ¹²⁶Sn umfasst.

7. Verfahren gemäß Anspruch 5 oder 6, wobei der durch beta-Partikel aktivierte Halbleiter und das Radionuklid gekoppelt werden, um einen radioaktiven Katalysator zu bilden.

8. Verfahren gemäß Anspruch 7, wobei der radioaktive Katalysator das Radionuklid in Kontakt mit dem Halbleiter mit hoher Bandlücke umfasst, das Radionuklid proximal zu dem Halbleiter mit hoher Bandlücke vorliegt, das Radionuklid physikalisch mit dem Halbleiter mit hoher Bandlücke vermischt ist, das Radionuklid chemisch in den Halbleiter mit hoher Bandlücke eingearbeitet ist, das Radionuklid in den Halbleiter mit hoher Bandlücke geladen ist und/oder der Halbleiter mit hoher Bandlücke außerhalb des Radionuklids angeordnet ist.

9. Verfahren gemäß Anspruch 7 oder 8, wobei der radioaktive Katalysator in der Form eines Partikels, eines Granulats, einer Perle, eines Pulvers, eines Pellets oder einer Fritte vorliegt.

10. System zum Umwandeln von CO₂ und/oder einer verwandten Form davon in eine oder mehrere kleine organische Verbindungen unter Verwendung des Verfahrens gemäß einem jeglichen der Ansprüche 1 bis 9, wobei das System umfasst:
eine Quelle für CO₂ und/oder eine verwandte Form davon;
einen Reaktionsbehälter, der einen radioaktiven Katalysator umfasst, der einen Halbleiter mit hoher Bandlücke und ein beta-Partikel emittierendes Radionuklid umfasst, zum Aussetzen gegenüber dem CO₂ und/oder der verwandten Form davon; und
Mittel zum Extrahieren einer oder mehrerer kleiner organischer Verbindungen, erzeugt durch Aussetzen des CO₂ und/oder der verwandten Form davon gegenüber dem radioaktiven Katalysator.

11. System gemäß Anspruch 10, wobei die Quelle für CO₂ und/oder eine verwandte Form davon eines oder mehrere aus Abfall-CO₂, atmosphärischem CO₂, flüssigem CO₂, sequestriertem CO₂, CO₂, das mit einem anderen Mittel komplexiert ist, einem Bicarbonat, einem Carbonat, einem Carbonaterz oder einer chemischen Verbindung, die CO₂ bereitstellt, umfasst.

12. Radiokatalytisches Material, umfassend einen Halbleiter mit hoher Bandlücke, der mit einem beta-Partikel emittierenden Radionuklid gekoppelt ist, wobei der Halbleiter eine Leitungsbandkantenenergie von weniger als -0,15 Volt, bezogen auf die Standard-Wasserstoffelektrode, aufweist.

## Revendications

1. Procédé de conversion de CO₂ et/ou d'une forme apparentée de celui-ci en un ou plusieurs petits composés organiques, le procédé comprenant l'exposition du CO₂ et/ou de la forme apparentée de celui-ci à un semi-conducteur à bande interdite élevée activé par particules bêta et de ce fait la conversion du CO₂ et/ou de la forme apparentée de celui-ci en un ou plusieurs petits composés organiques ;
lesdits un ou plusieurs petits composés organiques comprenant un ou plusieurs éléments parmi le formaldéhyde, le méthane, le méthanol, l'acide formique, l'éthanol, l'acétaldéhyde, l'acide acétique, le propanol et l'isopropanol ; et
ledit semi-conducteur possédant une énergie de bord de bande de conduction inférieure à -0,15 volts, par rapport à l'électrode à hydrogène standard.

2. Procédé selon la revendication 1, ledit semi-conducteur présentant une bande interdite d'au moins 2,6 eV.

3. Procédé selon la revendication 1 ou la revendication 2, ledit semi-conducteur comprenant un ou plusieurs parmi un titanate, un zirconate, un molybdate, un vanadate, un technétate, un pertechnétate, un tungstate, un niobate, un tantalate, un chromate, des oxydes d'étain dopés, un oxyde de zinc dopé, un hafnate, un oxyde de germanium, un oxyde simple, un oxyde de manganèse, de cobalt et de fer, un sulfure, un chalcogénure et un allotrope de carbone.

4. Procédé selon l'une quelconque des revendications 1 à 3, ledit semi-conducteur à bande interdite élevée activé par des particules bêta comprenant une activation par particules bêta par le biais d'une excitation continue par des particules bêta.

5. Procédé selon l'une quelconque des revendications 1 à 4, ledit semi-conducteur activé par particules bêta comprenant une activation par particules bêta par le biais d'une émission à partir d'un radionucléide.

6. Procédé selon la revendication 5, ledit radionucléide comprenant un ou plusieurs éléments parmi ¹⁴C, ⁹⁰Sr, ⁹⁹Tc, ³H, ⁶³Ni, ¹³⁷Cs, ¹⁴⁷Pm, ¹⁵¹Sm, ^{121m}Sn, ¹⁵⁵Eu, ⁹³Zr, ²¹⁰Pb et ¹²⁶Sn.

7. Procédé selon les revendications 5 ou 6, ledit semi-conducteur activé par particules bêta et ledit radionucléide étant couplés de manière à former un catalyseur radioactif.

8. Procédé selon la revendication 7, ledit catalyseur radioactif comprenant le radionucléide en contact avec le semi-conducteur à bande interdite élevée, le radionucléide à proximité du semi-conducteur à bande interdite élevée, le radionucléide mélangé physiquement avec le semi-conducteur à bande interdite élevée, le radionucléide incorporé chimiquement dans le semi-conducteur à bande interdite élevée, le radionucléide chargé dans le semi-conducteur à bande interdite élevée, et/ou ledit semi-conducteur à bande interdite élevée étant situé à l'extérieur du radionucléide.

9. Procédé selon la revendication 7 ou 8, ledit catalyseur radioactif se présentant sous la forme d'une particule, d'un granulé, d'une bille, d'une poudre, d'une pastille ou d'une fritte.

10. Système de conversion de CO₂ et/ou d'une forme apparentée de celui-ci en un ou plusieurs petits composés organiques à l'aide du procédé selon l'une quelconque des revendications 1 à 9, le système comprenant :
une source de CO₂ et/ou une forme apparentée de celui-ci ;
un récipient de réaction comprenant un catalyseur radioactif comprenant un semi-conducteur à bande interdite élevée et un radionucléide émetteur de particules bêta pour une exposition au CO₂ et/ou à la forme apparentée de celui-ci ; et
un moyen pour extraire un ou plusieurs petits composés organiques produits par exposition du CO₂ et/ou de la forme apparentée de celui-ci au catalyseur radioactif.

11. Système selon la revendication 10, ladite source de CO₂ et/ou forme apparentée de celui-ci comprenant une ou plusieurs sources parmi le CO₂ résiduaire, le CO₂ atmosphérique, le CO₂ liquide, le CO₂ séquestré, le CO₂ complexé avec un autre agent, un bicarbonate, un carbonate, un minerai de carbonate, ou un composé chimique qui fournit du CO₂.

12. Matériau radio-catalytique comprenant un semi-conducteur à bande interdite élevée couplé à un radionucléide émettant des particules bêta, ledit semi-conducteur possédant une énergie de bord de bande de conduction inférieure à -0,15 volts, par rapport à l'électrode à hydrogène standard.
